# EUROPÄISCHE PATENTANMELDUNG

(11) **EP 1 151 721 A2**
(43) Veröffentlichungstag der Anmeldung: **07.11.2001**
(21) Anmeldenummer: 01109573.4
(22) Anmeldetag: 18.04.2001
(51) Int. Cl.: A61B 5/103, A61B 1/04

(54) **Verfahren und Einrichtung zur Vermessung und Klassifizierung von optisch, einschliesslich endoskopisch beobachtbaren Haut- oder Schleimhaut-Veränderungen**

(30) Priorität: 03.05.2000 DE 10021431
(71) Anmelder: INB Vision AG, 39120 Magdeburg (DE)
(72) Erfinder: Michaelis, Bernd, Prof. Dr.-Ing., 39175 Biederitz (DE); Albrecht, Peter, 39104 Magdeburg (DE)
(74) Vertreter: Leinung, Günter

(57) **Zusammenfassung**

Die Erfindung betrifft ein Verfahren und eine Einrichtung zur Vermessung und Klassifizierung optisch beobachtbarer Veränderungen der Haut oder Schleimhaut, einschließlich endoskopisch beobachtbarer Veränderungen, in vorgegebene Kategorien.

Und das Verfahren ist gekennzeichnet durch einen ersten Bearbeitungsschritt, bei dem aus digitalen Bildern die Oberflächenmessdaten der Haut - oder Schleimhaut - Veränderungen berechnet verden, die aus den 3 D - Koordinaten der Veränderungen und den zugehörigen, auf das Referenzbild normierten Farbwerten, bestehen.
In einem zweiten Bearbeitungsschritt werden die Daten bewertet, wofür bevorzugt ein künstliches neuronales Netz verwendet wird.
Sollte eine sichere Klassifizierung nicht möglich sein, findet ein dritter Bearbeitungsschritt statt, der aus einem weiteren Klassifizierungsprozess besteht, der sich von dem ersten Klassifizierungsprozess durch einen modifizierten Algorhytmus unterscheidet und/oder der Verwendung einer Auswahl von in mindestens einer Datenbank gespeicherten Vergleichsdaten, insbesondere von durch Experten bewerteten Vergleichsdaten.

## Beschreibung

Die Erfindung betrifft ein Verfahren und eine Einrichtung zur Vermessung und Klassifizierung optisch beobachtbarer Veränderungen der Haut oder Schleimhaut, einschließlich endoskopisch beobachtbarer Veränderungen, in vorgegebene Kategorien.

Ein bevorzugtes Anwendungsgebiet der Erfindung ist die Klassifizierung von Hautanomalien wie Melanome und melanomähnliche Veränderungen bezüglich der Eigenschaften gutartig, bösartig oder verdächtig. Weitere Anwendungsgebiete sind die Klassifizierung von Anomalien von Häuten und Schleimhäuten endoskopisch beobachtbarer Hohlorgane, wie z.B. der Speiseröhre, des Magens, des Duodenums, des Colons, des Rektums, des Bronchialsystems, der Hohlräume des Hals-Nasen-Ohren- Bereiches und der Harnblase. Diese Anomalien sind z.B. Divertikel, Polypen, Geschwüre, Tumorinfiltrationen und Tumorkompressionen.

Maligne (bösartige) Melanome und dgl. sind derzeit erst in ihrer Spätphase mit hoher Sicherheit zu identifizieren, dann aber sehr gefährlich, in der Regel lebensgefährlich. In ihrer frühen Phase sind sie jedoch relativ einfach und erfolgreich behandelbar. In diesem Zeitabschnitt sind sie aber nur schwer identifizierbar; d.h. die Klassifizierung der Hautanomalien, insbesondere in maligne oder benigne (gutartige) Melanome ist schwierig. Die Aufgabe des behandelnden Arztes besteht somit in einer Klassifizierung von Hautanomalien in eine Gruppe von harmlosen Hautanomalien (benigne Melanome und andere gutartige nicht-melanozytäre Hautveränderungen) und eine Gruppe von eindeutig malignen bzw. für ihn verdächtigen Melanomen. Dabei können pro Person durchaus mehrere Hautanomalien begutachtet werden müssen. Um zu gewährleisten, daß kein einziges malignes Melanom übersehen wird, muß der behandelnde Arzt den Patienten beim geringsten Verdacht auf Malignität zur Gruppe der Weiterzubehandelnden zuordnen, um zu gewährleisten, daß kein einziges malignes Melanom übersehen wird.
Durch diese Vorgehensweise wird die Gruppe der Risikopatienten in der Regel sehr groß sein. Hieraus resultiert die Motivation, die Befundung durch Verwendung allgemein zugänglicher Hilfsmittel zu verbessern, um auch demjenigen Arzt eine qualitativ hochwertige Klassifizierung der Hautanomalien, insbesondere der Melanome, zu ermöglichen, der nicht über das notwendige Spezialwissen verfügt, um allein aufgrund visueller Informationen eine Klassifizierung durchzuführen.
Zur Erfassung von Anomalien der Haut, insbesondere von Melanomen, ist es bekannt, einen zweidimensionalen Untersuchungsbereich der Haut mit Licht eines ersten Wellenlängenbereiches zu beleuchten, der zur Anregung von Fluoreszenz geeignet ist, und im Wellenlängenbereich des Fluoreszenzlichtes ein Bild des Untersuchungsbereiches zu erzeugen (z. B. DE4026821 A1). Eine Auswertung des Fluoreszenzbildes allein ist jedoch für eine sichere Klassifizierung nicht ausreichend zuverlässig, weil auch die Topologie der Hautoberfläche im Untersuchungsbereich einen starken Einfluß auf die Intensitätsverteilung im Fluoreszenzbild besitzt. Gemäß DE4026821 A1 soll daher der Einfluß der Topologie der Hautoberfläche auf das erzeugte Bild reduziert werden. Hierzu wird der Untersuchungsbereich mit Licht eines zweiten Wellenlängenbereiches beleuchtet und ein Referenzbild mit Licht dieses zweiten Wellenlängenbereiches erzeugt. Indem man dann die Intensitätswerte im Fluoreszenzbild auf diejenigen des Referenzbildes normiert, erhält man ein zur Auswertung durch den behandelnden Arzt geeignetes Bild, in dem die von der Topologie bewirkten Intensitätsänderungen weitgehend eliminiert sind.

Aus der DE 19819516 A1 ist auch eine Fluoreszenzdiagnose-Einrichtung bekannt, die zur Tumor-Untersuchung der Innenwand von Körperhöhlen geeignet ist. Diese Einrichtung besteht im wesentlichen aus einem Endoskop und einer Kamera zur Ausgabe von Bildern des Untersuchungsbereiches auf einen Monitor sowie einem Spektrometer, um das von ausgewählten Stellen des Untersuchungsbereiches erzeugte Fluoreszenzlicht spektrometrisch zu untersuchen. Bei dieser Diagnoseeinrichtung erfolgt jedoch keine Bildverarbeitung, welche die Arbeit des untersuchenden Arztes erleichtern würde; außerdem bleibt die Topologie des untersuchten Gewebereiches ohne Einfluß auf die Diagnose.

Aus der W09747235 ist ein Diagnose-System und -Verfahren bekannt, das zur Quantifizierung der visuellen Erscheinung von Hautanomalien wie Melanomen geeignet ist, indem Abbildungen der Haut digital analysiert werden. Dieses System enthält in einer bevorzugten Ausführung eine hochauflösende Digitalkamera, eine Recheneinheit, einen Datenspeicher sowie einen Farbmonitor. Die Kamera erzeugt ein Bild des zu untersuchenden Hautbereiches sowie des Längenmaßstabes und der Farbskala, die in der Nähe des zu untersuchenden Hautbereiches angeordnet sind. Hierdurch ist es möglich, Farbwerte und Abstände im Bild unabhängig von den Beleuchtungs- und Aufnahmebedingungen zu normieren. Mittels der Recheneinheit wird die Außenkontur dunkler Hautgebiete bestimmt und nachfolgend auf dem Monitor angezeigt. Das Diagnosesystem erlaubt es auch, Kenngrößen für die Farbe des untersuchten Hautgebietes sowie zweidimensionale geometrische Kenngrößen der Außenkontur dunkler Hautgebiete wie Größe und Asymmetrie abzuleiten sowie die zu verschiedenen Zeitpunkten ermittelten Kenngrößen und Bilder zu vergleichen. Obwohl das Diagnose-System gem. W09747235 verschiedene geometrische Kenngrößen und Farbkenngrößen zur Verfügung stellt, ist die Identifizierung von Hautanomalien immer noch sehr stark von der Erfahrung des untersuchenden Arztes abhängig.

Um die Klassifizierung von Hautanomalien zu objektivieren und zu automatisieren, ist es bekannt, die Unterscheidungsmerkmale von Hautanomalien wie Form und Farbe in digitalen Farbbildern eines Hautbereiches für die Klassifizierung mittels eines neuronalen Netzes zu verarbeiten (Ercal, F.; et. al., IEEE Transactions on Biomedical Engineering, (1994 Sep.) 41 (9) 837-45). Das Verfahren stützt sich bei der Klassifizierung sehr wesentlich auf die Verarbeitung von Farbkenngrößen sowie auf die Asymmetrie und Unregelmäßigkeit des Randverlaufs der Hautanomalie. Das Verfahren verzichtet darauf, als Kenngröße im Klassifizierungsprozeß die Größe oder den Durchmessers der Hautanomalie bzw. deren zeitliche Änderungen zu nutzen, und erfordert eine sehr genaue Bestimmung der Ränder der Hautanomalien, die deshalb von einem Dermatologen manuell durchgeführt wird.

Ein anderes Beispiel für die Verwendung eines neuronalen Netzes zur Klassifizierung von Tumoren in Ultraschallbildern ist in US5260871 beschrieben. Hier werden charakteristische Merkmale aus den Ultraschallbildern extrahiert, die dann auf den Eingang des neuronalen Netzes gegeben werden, das eine Klassifizierung durchführt. Auch bei dieser Lösung werden keine Daten über die dreidimensionale Struktur des verdächtigen Gewebebereiches ausgewertet.

Ein wesentlicher Nachteil bekannter Lösungen besteht demnach darin, daß keine Daten über die dreidimensionale Struktur des verdächtigen Gewebebereiches ausgewertet und bei der Klassifizierung verwendet werden. Bei der Auswertung von Melanom-Untersuchungen hat sich jedoch gezeigt, daß die 3D-Struktur und das Höhenwachstum wichtige Kriterien zur Einschätzung des Charakters eines Melanoms sind.

Der Erfindung liegt somit die Aufgabe zugrunde, ein Verfahren und eine Einrichtung zu schaffen, die zur Vermessung und Klassifizierung von optisch beobachtbaren Haut- oder Schleimhaut-Veränderungen, insbesondere von Hautanomalien der Haut wie Melanomen und melanomähnlichen Erscheinungen geeignet sind und als Klassifizierungsmerkmal auch die dreidimensionale Struktur der Veränderungen und ihre zeitliche Variation berücksichtigen.

Diese Aufgabe wird mit einem Verfahren nach Anspruch 1 und einer Einrichtung nach Anspruch 10 gelöst.

Dem erfindungsgemäßen Verfahren liegt der Gedanke zugrunde, mittels geeigneter Aufnahmetechniken von den zu untersuchenden, optisch beobachtbaren Haut- oder Schleimhaut-Veränderungen in den für eine Diagnose geeigneten Zeitabständen solche digitalen Bilder zu erzeugen, die sowohl hinsichtlich der dreidimensionalen Struktur des abgebildeten Objekts als auch hinsichtlich seiner Farbstruktur auswertbar sind. Eine hierfür geeignete Aufnahmetechnik ist z. B. aus der DE 19623172 (Michaelis, B., Albrecht, P.: Verfahren zur dreidimensionalen optischen Vermessung von Objektoberflächen) bekannt.

In einem ersten Bearbeitungsschritt werden aus diesen digitalen Bildern die Oberflächenrneßdaten der Haut- oder Schleimhaut-Veränderungen berechnet, die aus den 3D-Koordinaten der Veränderungen und den zugehörigen, auf das Referenzbild normierten Farbwerten bestehen. Bedarfsweise werden zusätzlich die zeitlichen Änderungen dieser Daten berechnet.

In einem zweiten Bearbeitungsschritt werden diese Oberflächenmeßdaten durch einen Klassifizierungsprozeß bewertet, wofür bevorzugt ein künstliches neuronales Netz verwendet wird. Das Ergebnis ist eine Klassifizierung z. B. in benigne, verdächtige oder maligne Veränderungen.

Wenn auf der Basis des entsprechend dem erfindungsgemäßen Verfahren hergestellten Bildmaterials und der daraus abgeleiteten Oberflächenmeßdaten eine sichere Klassifizierung nicht möglich ist, findet nach einem weiteren Grundgedanken der Erfindung ein dritter Bearbeitungsschritt statt, der aus einem weiteren Klassifizierungsprozeß besteht, der sich von dem ersten Klassifizierungsprozeß durch einen modifizierten Algorithmus unterscheidet und/oder der Verwendung einer Auswahl von in mindestens einer Datenbank gespeicherten Vergleichsdaten, insbesondere von durch Experten bewerteten Vergleichsdaten.

In dieser Datenbank sind bewertete Krankheitsverläufe dokumentiert durch zu verschiedenen Zeitpunkten aufgenommene Bilder und 3D-Daten sowie für die Krankheit relevante Daten. Entscheidend ist, daß diese Krankheitsverläufe mit sehr hoher Sicherheit klassifiziert werden konnten, häufig durch histologische Untersuchungen. Statt der Klassifizierung der Haut- oder Schleimhaut-Veränderungen oder zusätzlich zur Klassifizierung kann ein mit der Datenbank verknüpftes künstliches neuronales Netz Vergleichsmaterial aus der Datenbank selektieren, d. h. Krankheitsverläufe mit Haut- oder Schleimhaut-Veränderungen, deren physikalische Eigenschaften und passende andere relevante Daten vergleichbar sind mit denen der zu untersuchenden Haut- oder Schleimhaut-Veränderungen. Aus den selektierten Vergleichsdaten können geeignete, den Eingangsdaten ähnliche Bilder ausgewählt werden und mit der Bewertung an den behandelnden Arzt zurückgeschickt werden. Diesem wird dadurch eine qualitativ bessere Klassifizierung ermöglicht.

Die Durchführung der Bearbeitungsschritte ist in folgenden Varianten möglich:
- alle Bearbeitungsschritte werden auf einer lokalen Recheneinheit durchgeführt,
- der erste und der zweite Bearbeitungsschritt werden auf einer lokalen Recheneinheit und der dritte Bearbeitungsschritt auf einer zentralen Recheneinheit durchgeführt,
- der erste Bearbeitungsschritt wird auf einer lokalen Recheneinheit und der zweite sowie dritte Bearbeitungsschritt werden auf einer zentralen Recheneinheit durchgeführt,
- alle Bearbeitungsschritt werden unter Verwendung einer zentralen Recheneinheit durchgeführt.

Nach einer anderen vorteilhaften Ausführungsform des erfindungsgemäßen Verfahrens wird mindestens eine der Datenbanken, die von durch Experten bewertete Vergleichsdaten enthält, auf der zentralen Recheneinheit bereitgestellt und gepflegt, wobei die lokale Recheneinheit einen Zugriff hat auf diese Datenbank besitzt.

Die erfindungsgemäße Einrichtung besteht aus zwei Hauptkomponenten: Einem Meßkopf zur Erzeugung von mindestens zwei photogrammetrisch auswertbaren Bildern der zu untersuchenden und zu klassifizierenden Haut- oder Schleimhaut-Veränderungen und mindestens einer Recheneinheit, die an den Meßkopf über Datenleitungen angeschlossenen sind oder von denen mindestens eine direkt in den Meßkopf integriet ist.
Der Meßkopf enthält mindestens zwei kalibrierte Kameras zur Erzeugung digitaler Bilder des zu untersuchenden Bereiches, nachfolgend als Kameragruppe bezeichnet, und einen Musterprojektor, der auf den zu untersuchenden Bereich mindestens ein zur Ermittlung von 3D-Daten des genannten Bereiches geeignetes Muster projiziert. Die Kameragruppe ist so justiert, daß mit ihnen eine Fläche geeigneter Größe vermessen werden kann. Mindestens eine der Kameras der Kameragruppe ist zur Aufnahme von Farbbildern geeignet, wobei diese Kamera zusätzlich oder gegebenenfalls eine weitere Kamera zur Aufnahme eines unauffälligen Bereiches in der Umgebung der Haut- oder Schleimhaut-Veränderung als Referenzbild für die Bestimmung einer Referenzfarbe vorgesehen ist. Der Meßkopf ist beweglich und kann an eine geeignete Stelle positioniert werden.

Die an den Meßkopf über Datenleitungen angeschlossene mindestens eine Recheneinheit weist Mittel auf, um die vom Meßkopf erzeugten digitalen Bilder der Haut- oder Schleimhaut-Veränderungen aufzubereiten, zu verarbeiten und zu speichern, insbesondere Mittel zur Berechnung von Daten der drei- und zweidimensionalen Strukturen der Veränderungen und ihrer Farbstrukturen in Bezug auf das Referenzbild sowie der zeitlichen Änderungen der genannten Daten. Diese Recheneinheit weist ferner Mittel auf, um aus den - vorstehend angegebenen - ermittelten Daten und gegebenenfalls zusammen mit den in einer Patientendatenbank gespeicherten, relevanten patientenbezogenen Daten wie Alter, Hauttyp, Allergien, dermatoskopische oder sonografische Befunde usw. eine Klassifizierung der Haut- oder Schleimhaut-Veränderungen durchzuführen. Für diese Klassifizierung kann z. B. ein neuronales Netz verwendet werden.

Vorzugsweise zur Anwendung in einem nicht sicher klassifizierbaren Fall weist die Recheneinheit Mittel auf, um auf der Basis der vorher ermittelten Daten und gegebenenfalls der in der Patientendatenbank gespeicherten relevanten patientenbezogenen Daten eine weitere Klassifizierung mit einem modifizierten Algorithmus und/oder eine Auswahl aus mindestens einer Datenbank mit von Experten bewerteten Vergleichsdaten durchzuführen.

In einer vorteilhaften Ausführungsform der erfindungsgemäßen Einrichtung sind mindestens zwei mittels Datenleitungen verbundene Recheneinheiten vorgesehen. Davon ist mindestens eine Recheneinheit als lokale Recheneinheit und mindestens eine Recheneinheit als zentrale Recheneinheit ausgeführt. In diesem Fall weist ausschließlich die zentrale Recheneinheit die in einem nicht sicher klassifizierbaren Fall zur Anwendung gelangenden Mittel auf, insbesondere die Datenbank mit von Experten bewerteten Vergleichsdaten.

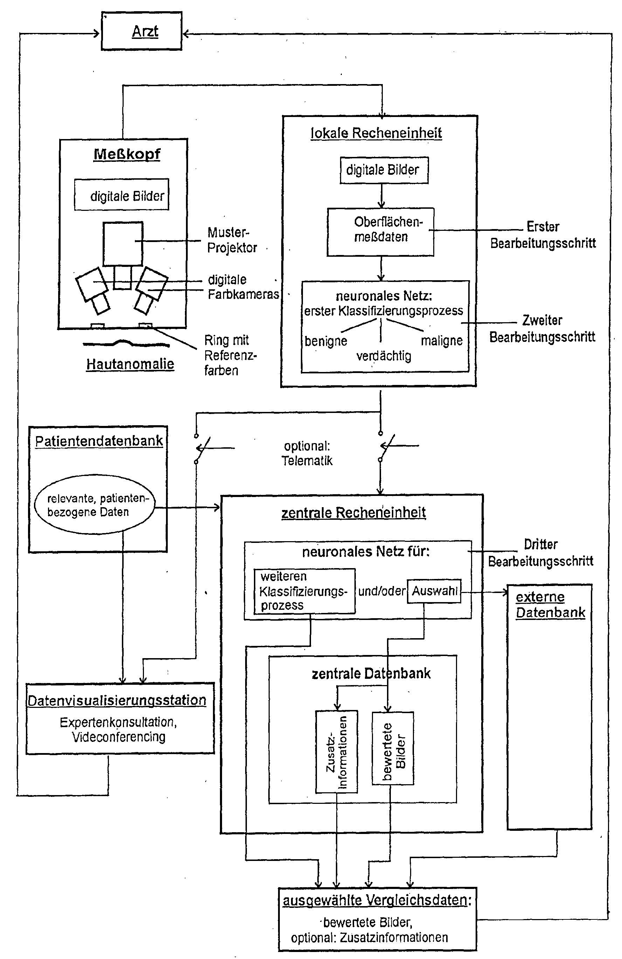

Im folgenden wird beispielhaft eine typische Ausführungsform der Erfindung zur Klassifizierung von Hautanomalien unter Bezugnahme auf die Zeichnung beschrieben, in der das Zusammenwirken der erfindungsgemäßen Verfahrensschritte als grafisches Schema dargestellt ist.

Dieses Beispiel betrifft die Klassifikation von Hautanomalien, die als Melanome und melanozytäre Veränderungen bekannt sind, in geeignete Kategorien, wie z.B. benigne, verdächtig oder maligne.

Der Meßkopf enthält eine aus zwei kalibrierten Farbkameras bestehende Kameragruppe zur Erzeugung digitaler Bilder und einen Musterprojektor. Der Musterprojektor ermöglicht bedarfsweise die Beleuchtung der Hautanomalien mit unstrukturiertem Licht, d. h. mit einer im wesentlichen konstanten Beleuchtungsstärke, mit einem Licht-Muster oder mit mehreren, zeitlich nacheinander folgenden Licht-Mustern. Die Objektive und die Positionen der Kameragruppe und des Musterprojektors werden so gewählt, daß mit ihnen eine Fläche von ca. 4×4 cm² vermessen werden kann. Ein weiterer Bestandteil des Meßkopfes ist eine Fläche (z.B. ein Ring) mit Referenzfarben. Diese dient zum Abgleich der Farb-Meßdaten der Kameragruppe. Dies ist sehr sinnvoll, um Meßergebnisse vergleichen zu können, die mit verschiedenen Meßköpfen erzeugt werden.

Um die notwendigen Bilder zur Bestimmung der Meßdaten der Hautanomalien zu gewinnen, eignet sich folgende Vorgehensweise:
Zunächst wird ein Farbbild eines gesunden Hautbereichs in der Nähe der Hautanomalie aufgenommen. Aus diesem Referenzbild wird die aktuelle Basishautfarbe des Patienten (Grundkomplexion) bestimmt. In Relation zu dieser können die Farbinformationen der Hautanomalie normiert werden.
Anschließend wird der Meßkopf so positioniert, daß sich die Hautanomalie möglichst zentral im Meßbereich der Kameragruppe befindet. Beim Vorliegen von zwei oder mehreren, dicht beieinander liegenden Hautanomalien kann es sinnvoll sein, von dieser Regel abzuweichen, so daß diese Hautanomalien eventuell durch eine einzige Position des Meßkopfes erfaßt werden. Nun kann durch mindestens eine Kamera der Kameragruppe ein als sog. Hellbild bezeichnetes Farbbild aufgenommen werden, bei dem die Hautanomalie mit unstrukturiertem Licht beleuchtet wird. Aus diesem Hellbild werden die aktuellen Farbwerte der Hautveränderung bestimmt.
Des weiteren werden bei unveränderter Lage des Meßkopfes durch die Kameragruppe Bilder aufgenommen, bei denen in der Regel ein oder zeitlich nacheinander mehrere geeignete Licht-Muster mit Hilfe des Musterprojektors auf die Hautanomalie projiziert werden. Diese Licht-Muster werden verfahrensbedingt benötigt.

Die aufgenommenen digitalen Bilder bzw. Bildfolgen sind Grundlage für die Berechnung der Daten der drei- und zweidimensionalen Strukturen der Hautanomalie und ihrer Farbstrukturen in Bezug auf das Referenzbild. Zu diesem Zweck berechnet eine lokale Recheneinheit, ausgehend von den aufgenommenen, digitalen Bildern, in einem ersten Bearbeitungsschritt Oberflächenmeßdaten der Hautanomalie, die aus den 3D-Koordinaten und den zugehörigen normierten Farbwerten der Oberfläche der Hautanomalie bestehen.

Zur Bestimmung der 3D-Koordinaten werden die Bilder bzw. die Bildfolgen der Hautanomalie verwendet, bei deren Aufnahme Licht-Muster projiziert wurden. Für die Berechnung werden bekannte oder neuartige Algorithmen aus der Bildverarbeitung verwendet. Diese basieren auf der Bestimmung korrespondierender Punktepaare durch Matchingverfahren und Korrelationsalgorithmen.
Zur Bestimmung der normierten Farbwerte der Hautanomalie werden das Hellbild und das Referenzbild benutzt. Die Farbwerte des Hellbildes werden durch die Aktivität der Hautanomalie aber auch durch die Grundkomplexion der Haut (abhängig vom jahreszeitlich bedingten Bräunungsgrad) beeinflusst. Um letztgenannten Einfluß auf eine Klassifizierung zu minimieren, ist eine Normierung in Bezug auf das Referenzbild sinnvoll. Diese Normierung kann auf klassische Weise (Verwendung eines Offsets und einer Helligkeitskorrektur) oder durch ein neuronales Netz erfolgen. Das Ergebnis sind die normierten Farbwerte. Die Zuordnung der normierten Farbwerte zu den 3D-Koordinaten ist einfach, da beide Datenmengen aus den Bildern denselben Kameras bei gleicher Position bestimmt werden.

Die auf diese Weise bestimmten Oberflächenmeßdaten der Hautanomalie werden in einem zweiten Bearbeitungsschritt, der ebenfalls auf der lokalen Recheneinheit ausgeführt wird, mittels eines künstlichen neuronalen Netzes einem ersten Klassifizierungsprozeß unterworfen. Das Ergebnis ist eine Zuordnung zu geeigneten Kategorien, z.B. benigne, maligne oder verdächtig. Dieses Ergebnis und die gewonnenen Oberflächenmeßdaten eignen sich gut für eine Dokumentation und Archivierung in einer Patientendatenbank. Werden Messungen zu verschiedenen Zeitpunkten gemacht, dann können aus deren Dokumentation auch die relativen Änderungen der Oberflächenmeßdaten der Hautanomalie bestimmt werden.

Zur weiteren Erhöhung der Qualität der Klassifizierung kann optional eine Telematikanwendung aktiviert werden. Hierbei werden die Oberflächenmeßdaten der vom behandelnden Arzt vermessenen Hautanomalie an eine zentrale Recheneinheit übertragen sowie anderweitig erhobene, relevante patientenbezogene Daten wie Alter, Hauttyp, Allergien, dermatoskopische oder sonografische Befunde usw.
Auf der zentralen Recheneinheit kann dann der dritte Bearbeitungsschritt ausgeführt werden: ein weiterer Klassifizierungsprozeß basierend auf modifizierten Algorithmen und/oder eine Auswahl von geeigneten Vergleichsdaten aus mindestens einer Datenbank.
Diese Datenbank kann in Form einer zentralen Datenbank Bestandteil der zentralen Recheneinheit sein. Alternativ oder zusätzlich kann diese Datenbank auch als externe Datenbank ausgebildet sein, die z.B. über das Internet zugänglich ist.
Diese Datenbanken enthalten bewertete Krankheitsverläufe, die durch zu verschiedenen Zeitpunkten aufgenommene Bilder und berechnete Oberflächenmeßdaten sowie für die Krankheit relevante, patientenbezogene Daten (wie bereits beschrieben)dokumentiert sind. Entscheidend ist, daß diese Krankheitsverläufe mit sehr hoher Sicherheit klassifiziert werden konnten, häufig durch histologische Untersuchungen.

Aus den genannten Datenbanken detektiert das künstliche neuronale Netz oder ein anderer geeigneter Algorithmus der zentralen Recheneinheit Hautanomalien, deren Oberflächenmeßdaten eine ausreichende Ähnlichkeit aufweisen zu der vom behandelnden Arzt vermessenen Hautanomalie, deren Daten an die zentralen Recheneinheit übertragen wurden. Dabei werden auch die zugehörigen patientenbezogenen Daten berücksichtigt. Dadurch können geeignete Vergleichsdaten erhaltenen werden. Diese sind bewertete Bilder und optional Zusatzinformationen, wie z.B. der histologische Befund und andere interessierende Daten aus dem Krankheitsverlauf. Sie werden an den behandelnden Arzt zurückgeschickt. Mit ihnen kann das Klassifizierungsergebnis verbessert werden.

Eine andere telematische Anwendung besteht in der Konsultation eines Experten, dem die Oberflächenmeßdaten, das Klassifizierungsergebnis und die relevanten patientenbezogenen Daten per Datenleitungen zur Verfügung gestellt wurden. Diese Konsultation kann als Videoconferencing durchgeführt werden.

Weitere Anwendungsmöglichkeiten des erfindungsgemäßen Verfahrens und der Einrichtung bestehen bei folgenden Problemstellungen:
- Der Meßkopf kann auch zur Diagnoseunterstützung für andere Erkrankungen in der Dermatologie verwendet werden: Ein typischer Anwendungsfall ist die Vermessung und Bewertung von Flechten. Eine weitere Anwendung ist die Vermessung von Farb- und Höhenänderungen hervorgerufen durch Allergietests. Durch Verwendung des Meßkopfes ist eine präzise Dokumentation möglich.
- Zur Dokumentation und Einschätzung des Heilungsvorganges von Wunden ist eine Vermessung sehr sinnvoll. Hierbei werden sowohl 2D- und 3D-Daten als auch die farbliche Struktur zu einem oder mehreren Zeitpunkten gemessen. Ähnliches gilt für Verbrennungen, bei denen es außerdem auf die Bestimmung des Verbrennungsgrades ankommt.
- Bei Bestrahlungen von Patienten kann es ebenfalls zu Farbänderungen der Haut kommen. Werden diese gemessen, dann können Rückschlüsse auf die Strahlungsempfindlichkeit gezogen werden.
- Zur Vermessung und Bewertung von Hautfalten (vor allem im Gesicht) kann der Meßkopf ebenfalls eingesetzt werden. Hierfür gibt es einen Bedarf in der Kosmetikindustrie. Es sollen Rückschlüsse auf die Wirksamkeit von Hautcremes gezogen werden.

## Patentansprüche

1. Verfahren zur Vermessung und Klassifizierung von optisch, einschließlich endoskopisch beobachtbaren Haut- oder Schleimhaut-Veränderungen, insbesondere von Anomalien der Haut wie Melanome und melanomähnliche Erscheinungen, in vorgegebene Kategorien, bei dem
1. in einem oder mehreren Zeitpunkten digitale Bilder der Haut- oder Schleimhaut-Veränderungen, die zur Bestimmung der dreidimensionalen Struktur und der Farbstruktur der Veränderungen geeignet sind, und ein oder mehrere digitale Referenzbilder mindestens einer Umgebung der Veränderungen aufgenommen und bedarfsweise gespeichert werden,
2. in einem ersten Bearbeitungsschritt aus diesen digitalen Bildern die Oberflächenmeßdaten der Haut- oder Schleimhaut-Veränderungen berechnet werden, die aus den 3D-Koordinaten der Veränderungen und den zugehörigen, auf das Referenzbild normierten Farbwerten bestehen, sowie bedarfsweise zusätzlich die zeitlichen Änderungen dieser Daten,
3. in einem zweiten Bearbeitungsschritt mit den gem. Ziff. 2 ermittelten Oberflächenmeßdaten gegebenenfalls zusammen mit anderweitig erhobenen, relevanten patientenbezogenen Daten wie Alter, Hauttyp, Allergien, dermatoskopische oder sonografische Befunde usw. ein erster Klassifizierungsprozeß durchgeführt wird, bevorzugt durch ein neuronales Netz,
4. optional, vorwiegend in einem nicht sicher klassifizierbaren Fall, ein dritter Bearbeitungsschritt stattfindet, der aus einem weiteren Klassifizierungsprozeß besteht, der sich von dem ersten Klassifizierungsprozeß durch einen modifizierten Algorithmus unterscheidet und/oder der Verwendung einer Auswahl von in mindestens einer Datenbank gespeicherten Vergleichsdaten, insbesondere von durch Experten bewertete Vergleichsdaten, wobei in diesem dritten Bearbeitungsschritt die in Ziff. 2 ermittelten Oberflächenmeßdaten und optional die relevanten patientenbezogenen Daten sowie das in Ziff. 3 erhaltene Klassifikationsergebniss verwendet werden,
5. und die mit dem zweiten und dritten Bearbeitungsschritt erhaltenen Klassifikationsergebnisse ausgegeben werden.

2. Verfahren nach Anspruch 1, **dadurch gekennzeichnet, daß** bei der Aufnahme der digitalen Bilder, die zur Bestimmung der dreidimensionalen Struktur der Haut- oder Schleimhaut-Veränderungen verwendet werden, ein oder zeitlich nacheinander mehrere Licht-Muster auf die Veränderungen projiziert werden.

3. Verfahren nach Anspruch 1 oder 2, **dadurch gekennzeichnet, daß** bei der Aufnahme der digitalen Bildern, die zur Bestimmung der Farbstruktur der Haut- oder Schleimhaut-Veränderungen verwendet werden, mit unstrukturiertem Licht beleuchtet wird.

4. Verfahren nach einem der Ansprüche 1 bis 3, **dadurch gekennzeichnet, daß**
• alle Bearbeitungsschritte auf einer lokalen Recheneinheit durchgeführt werden oder
• der erste und der zweite Bearbeitungsschritt auf einer lokalen Recheneinheit und der dritte Bearbeitungsschritt auf einer zentralen Recheneinheit durchgeführt werden oder
• der erste Bearbeitungsschritt auf einer lokalen Recheneinheit und der zweite sowie dritte Bearbeitungsschritt auf einer zentralen Recheneinheit durchgeführt werden oder
• alle Bearbeitungsschritt unter Verwendung einer zentralen Recheneinheit durchgeführt werden.

5. Verfahren nach Anspruch 1 und 4, **dadurch gekennzeichnet, daß** mindestens eine der Datenbanken mit Vergleichsdaten auf der zentralen Recheneinheit bereitgestellt und gepflegt wird.

6. Verfahren nach Anspruch 1 und 4, **dadurch gekennzeichnet, daß** mindestens eine der Datenbanken mit Vergleichsdaten auf der lokalen Recheneinheit bereitgestellt wird.

7. Verfahren nach Anspruch 1 und 4, **dadurch gekennzeichnet, daß** mindestens eine der Datenbanken mit Vergleichsdaten auf einer externen Recheneinheit bereitgestellt und gepflegt wird, die weder die lokale noch die zentrale Recheneinheit ist.

8. Verfahren nach einem der vorhergehenden Ansprüche, **dadurch gekennzeichnet, daß** die lokale Recheneinheit einen Zugriff hat auf die Datenbanken mit den von Experten bewerteten Vergleichsdaten, die auf der zentralen Recheneinheit und/oder der externen Recheneinheit bereitgestellt werden.

9. Verfahren nach einem der vorhergehenden Ansprüche, **dadurch gekennzeichnet, daß** die Oberflächenmeßdaten der Haut- oder Schleimhaut-Veränderungen und die relevanten patientenbezogenen Daten zur visuellen Auswertung an eine externe Datenvisualisierungsstation übermittelt werden.

10. Einrichtung zur Vermessung und Klassifizierung von optisch, einschließlich endoskopisch beobachtbaren Haut- oder Schleimhaut-Veränderungen, insbesondere von Anomalien der Haut wie Melanome und melanomähnliche Erscheinungen, in vorgegebene Kategorien, bestehend aus
1. einem Meßkopf zur Erzeugung von mindestens zwei photogrammetrisch auswertbaren Bildern von Haut- oder Schleimhaut-Veränderungen, enthaltend mindestens zwei kalibrierte Kameras zur Erzeugung digitaler Bilder des zu untersuchenden Haut- oder Schleimhaut-Bereiches, nachfolgend als Kameragruppe bezeichnet, und einen Musterprojektor, der auf den zu untersuchenden Haut- oder Schleimhaut-Bereich mindestens ein zur Ermittlung von 3D-Daten des genannten Bereiches geeignetes Muster projiziert, wobei mindestens eine der Kameras der Kameragruppe zur Aufnahme von Farbbildern geeignet ist und diese zur Aufnahme von Farbbildern geeignete Kamera zusätzlich oder gegebenenfalls eine weitere Kamera vorgesehen ist, um einen gesunden Haut- oder Schleimhaut-Bereich in der Umgebung der Haut- oder Schleimhaut-Veränderung als Referenzbild für die Bestimmung einer Referenzfarbe aufzunehmen,
2. einer ersten Recheneinheit, um die vom Meßkopf erzeugten digitalen Bilder der Haut- oder Schleimhaut-Veränderungen aufzubereiten, zu verarbeiten und zu speichern, insbesondere zur Berechnung von Oberflächenmeßdaten der Veränderungen, die aus den 3D-Koordinaten der Veränderungen und den zugehörigen, auf das Referenzbild normierten Farbwerten bestehen, sowie zur Berechnung der zeitlichen Änderungen dieser Daten,
3. einer zweiten Recheneinheit, um mit den ermittelten Oberflächenmeßdaten und gegebenenfalls zusammen mit den in einer Patientendatenbank gespeicherten relevanten patientenbezogenen Daten wie die Alter, Hauttyp, Allergien, dermatoskopischen oder sonografischen Befunden usw. einen ersten Klassifizierungsprozeß der Veränderungen durchzuführen,
4. sowie optional einer weiteren Recheneinheit, vorzugsweise in einem nicht sicher klassifizierbaren Fall, um auf der Basis der Oberflächenmeßdaten und gegebenenfalls der in der Patientendatenbank gespeicherten relevanten patientenbezogenen Daten einen weiteren Klassifizierungsprozeß mit einem modifizierten Algorithmus und/oder eine Auswahl aus mindestens einer Datenbank mit von Experten bewerteten Vergleichsdaten durchzuführen.

11. Einrichtung nach Anspruch 10, **dadurch gekennzeichnet, daß** die erste Recheneinheit für die Meßdatenverarbeitungund die zweite Recheneinheit für die erste Klassifizierung in einer einzigen Recheneinheit für Meßdatenverarbeitung und erste Klassifizierung zusammengefaßt sind.

12. Einrichtung nach Anspruch 10 oder 11, **dadurch gekennzeichnet, daß**
• alle Recheneinheiten als lokale Recheneinheiten ausgebildet sind oder
• die Recheneinheiten für Meßdatenverarbeitung und erste Klassifizierung als lokale Recheneinheit ausgeführt sind und die Recheneinheit für den weiteren Klassifizierungsprozeß mit einem modifizierten Algorithmus und/oder eine Auswahl aus mindestens einer Datenbank mit von Experten bewerteten Vergleichsdaten als zentrale Recheneinheit ausgebildet sind oder
• die Recheneinheit für die Meßdatenverarbeitung als lokale Recheneinheit ausgeführt ist und die Recheneinheiten für die erste Klassifizierung sowie die Recheneinheit für den weiteren Klassifizierungsprozeß mit einem modifizierten Algorithmus und/oder eine Auswahl aus mindestens einer Datenbank mit von Experten bewerteten Vergleichsdaten als zentrale Recheneinheiten ausgebildet sind oder
• alle Recheneinheiten als zentrale Recheneinheiten ausgebildet sind.

13. Einrichtung nach Anspruch 12, **dadurch gekennzeichnet, daß** die zentrale Recheneinheit mindestens eine der Datenbanken mit Vergleichsdaten aufweist.

14. Einrichtung nach Anspruch 12, **dadurch gekennzeichnet, daß** die lokale Recheneinheit mindestens eine der Datenbanken mit Vergleichsdaten aufweist.

15. Einrichtung nach Anspruch 13, **dadurch gekennzeichnet, daß** mindestens eine Recheneinheit vorgesehen ist, die weder die lokale noch die zentrale Recheneinheit ist und mindestens eine der Datenbanken mit Vergleichsdaten aufweist

16. Einrichtung nach einem der vorhergehenden Ansprüche, **dadurch gekennzeichnet, daß** mindestens eine Recheneinheit über Datenleitungen mit mindestens einer entfernten Datenvisualisierungsstation verbunden ist.

17. Einrichtung nach einem der vorhergehenden Ansprüche, **dadurch gekennzeichnet, daß** der Meßkopf und die lokale Recheneinheit eine bauliche Einheit bilden.

18. Einrichtung nach Anspruch 10, **dadurch gekennzeichnet, daß** sie zur Vermessung ausschließlich aus dem Meßkopf und der ersten Recheneinheit für die Meßdatenverarbeitung besteht.
